# EUROPEAN PATENT APPLICATION

(11) **EP 4 656 185 A1**
(43) Date of publication of application: **03.12.2025**
(21) Application number: 24758689.4
(22) Date of filing: 09.07.2024
(51) Int. Cl.: A61K 9/14, A61K 31/4439, A61P 35/00

(54) **NOVEL MOLECULAR ASSEMBLY OF AXITINIB**

(30) Priority: 12.04.2024 KR 20240049313
(71) Applicant: Scai Therapeutics Co., Ltd., Seoul 06524 (KR)
(72) Inventor: KIM, Chul Hwan, Seo-gu, Daejeon 35249 (KR); KIM, Kyoung Hee, Seo-gu, Daejeon 35249 (KR)
(74) Representative: dompatent
(86) International application number: PCT/KR2024/009769
(87) International publication number: WO 2025/216362

(57) **Abstract**

The present invention relates to a molecular association, which is a novel axitinib polymorph in which axitinib is physically bonded.

## Description

### [Technical Field]

The present invention relates to a novel molecular association of axitinib.

### [Background Art]

Axitinib is a tyrosine kinase inhibitor, which is a compound with the structure of 6-[2-(methylcarbamoyl)phenylsulfanyl]-3-E-[2-(pyridin-2-yl)ethenyl]indazole represented by following Formula 1, and is well known as the main ingredient in Pfizer's Inlyta^{®}, which is a therapeutic agent for kidney cancer.

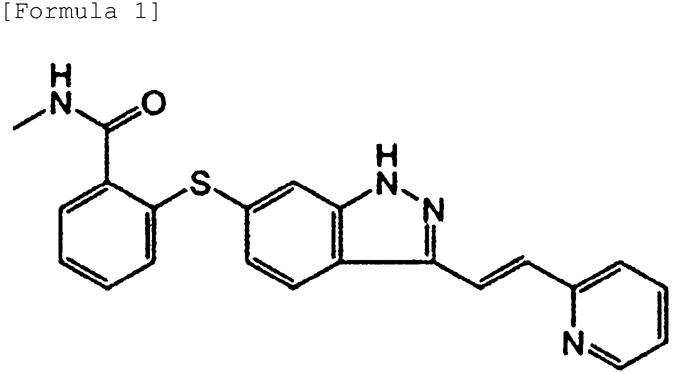

Axitinib is known to exist in several polymorphs, but the known polymorphs have the problem of being thermodynamically unstable or photochemically unstable, and thus, new polymorphs are continuously being studied.

For example, Pfizer stated in 2006 that Form IV (U.S. Patent Application Laid-Open No. US 2006/0094763 A1) is thermodynamically most stable polymorph of axitinib, but later introduced Forms XXV and XLI (European Patent No. EP 2134702 B2) and introduced Forms XXV and XLI as a more thermally stable form than Form IV in terms of density, heat of fusion and solubility. In addition, Forms XXV and XLI are said to have the advantage of improved photostability, a more regular crystal form, no tendency to form aggregates, and bulk flow properties that do not stick to probes in tanks. It is said that these improved properties made it easier to process and prepare tablets, and the filtering process took a long time of 26 hours when preparing Form IV, but the filtering time was shortened to 4 hours when preparing Forms XXV and XLI. In addition, it is said that since ethanol was used in the preparation of Forms XXV and XLI, problems caused by the lower flash point and toxicity problems did not occur compared to the preparation process of Form IV using n-heptane.

In this way, new polymorphs of axitinib taking into account the preparation process and stability are being continuously studied.

### [Prior Art Documents]

### [Patent Documents]

(Patent Document 1) US 2006/0094763 A1
(Patent Document 2) EP 2134702 B2

### [Disclosure]

### [Technical Problem]

The present invention introduces a molecular association in which axitinib is physically bonded, which not only facilitates the preparation process but also has excellent solubility and stability.

Therefore, it is an object of the present invention to provide a novel molecular association of axitinib that is easy to prepare and has excellent solubility and stability, and a preparation process thereof.

### [Technical Solution]

In order to achieve the above object,
the present invention provides a molecular association in which axitinib is physically bonded,
wherein the X-ray powder diffraction spectrum of the molecular association has X-ray diffraction peaks at diffraction angles 2θ of 24.99°±0.1° and 26.32°±0.1°.

In addition, the molecular association of the present invention may have a differential scanning calorimetry (DSC) profile characterized by a glass transition at a single endothermic temperature of 220.4±2.0 °C when measured under DSC conditions of 10 °C/min heating rate, 99.999% N2 and 30 to 250 °C.

In addition, the molecular association of the present invention may have an average particle diameter of 3 to 12 µm.

In addition, the molecular association of the present invention may have the following solubility: a dissolution concentration at pH 1 of 3.0 mg/mL or more and a dissolution concentration at pH 2 of 0.1 mg/mL or more.

### [Advantageous Effects]

The novel molecular association of axitinib according to the present invention has the advantage of having superior solubility and stability compared to conventional general axitinib.

In addition, a pharmaceutical composition comprising the molecular association of axitinib of the present invention has the advantage of being easily dissolved and absorbed and having excellent bioavailability.

### [Description of Drawings]

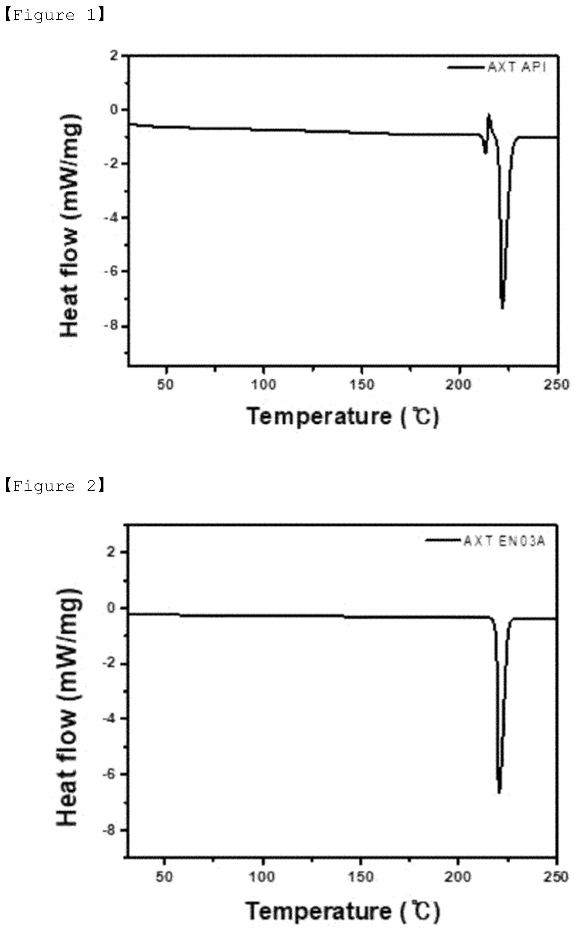

Figure 1 shows a DSC of Axitinib API, which is Comparative Example 1.
Figure 2 shows a DSC of Axitinib SCAI-Form, which is Example 1 of the present invention.

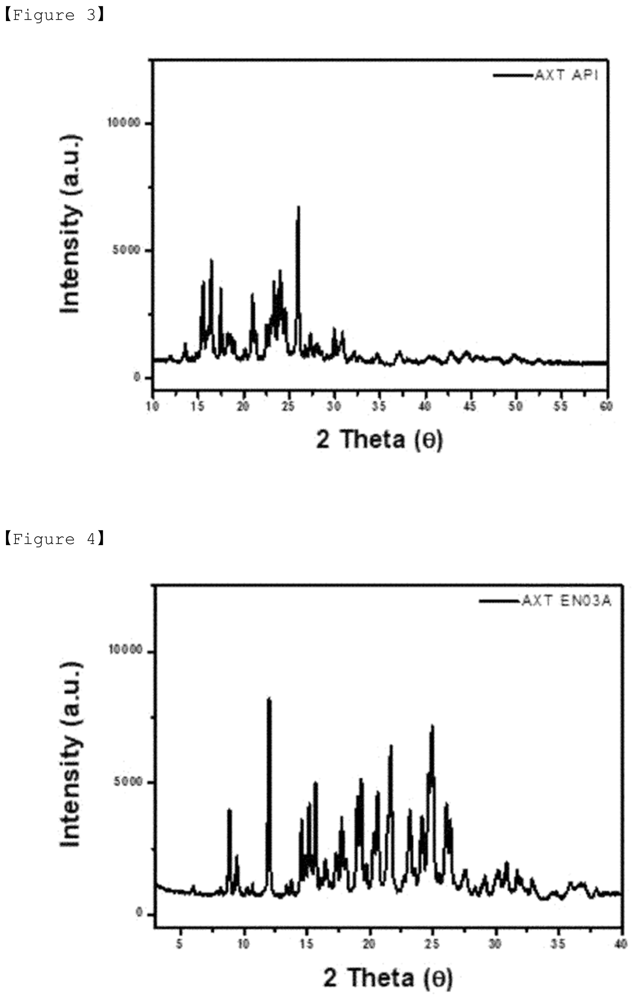

Figure 3 shows an XRD of Axitinib API, which is Comparative Example 1.
Figure 4 shows an XRD of Axitinib SCAI-Form, which is Example 1 of the present invention.
Figure 5 shows a SEM image of Axitinib API, which is Comparative Example 1.
Figure 6 shows SEM images of Axitinib SCAI-Form, which is Example 1 of the present invention.

### [Best Mode]

### Terms

As used herein, the term "precursor" refers to a precursor substance used to produce axitinib according to the present invention. That is, the precursor of axitinib according to the present invention refers to axitinib or a salt of axitinib to which shear stress is not applied.

As used herein, the term "molecular association" refers to a molecular association in which axitinib is physically bonded, and when formed as a composition comprising the molecular association in water, the molecular association in the composition may have an aggregated structure.

As used herein, the term "aspect ratio" refers to the length of a particle divided by the thickness of the particle. The "length of particle" refers to the largest diameter among the diameters of a particle measured in the present invention. The "thickness of particle" refers to the shortest diameter among the diameters of a particle measured in the present invention. Therefore, the aspect ratio is calculated as the ratio of these.

The molecular association refers to a molecular association in which axitinib is physically bonded, and when formed as a composition comprising the molecular association in water, the molecular association in the composition may have an aggregated structure.

### Axitinib of the present invention

The present invention provides a molecular association in which axitinib, which is a compound of following Formula 1, is physically bonded.

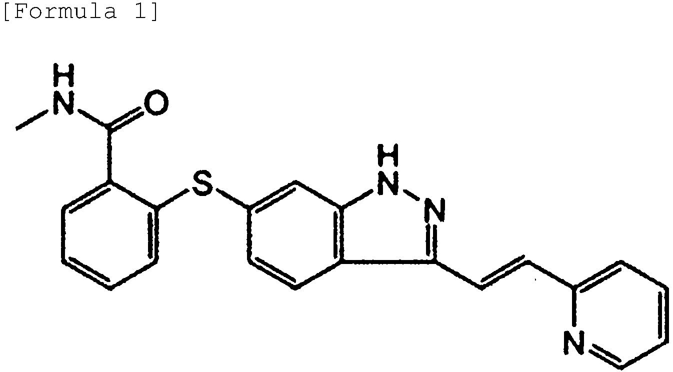

The X-ray powder diffraction spectrum of the molecular association in which the axitinib is physically bonded has X-ray diffraction peaks at diffraction angle 2θ of 24.99°±0.1° and 26.32°±0.1°.

In addition, the molecular association in which axitinib is physically bonded according to the present invention may have a differential scanning calorimetry (DSC) profile with a glass transition at a single endothermic temperature of 220.4±2.0 °C when measured under DSC conditions of 10 °C/min heating rate, 99.999% N2 and 30 to 250 °C. That is, while conventional general axitinib exhibits a glass transition at two endothermic temperatures of about 212.5 °C and about 220.6 °C based on the DSC profile, the molecular association in which axitinib is physically bonded according to the present invention has a difference in that it has a DSC profile characterized by a glass transition at a single endothermic temperature of 220.4±2.0 °C.

In addition, the molecular association in which axitinib is physically bonded according to the present invention may have an average particle diameter of 2.0 to 15 µm, and preferably, may have an average particle diameter of 3.0 µm or more, 5.0 µm or more, and may have an average particle diameter of 13.0 µm or less, 10.0 um or less. When the average particle diameter of the molecular association exceeds 15.0 µm, there are problems that dispersibility is poor and transparency and permeability are poor. In addition, when the average particle diameter of the molecular association is less than 2.0 µm, there are problems that it is difficult to prepare and performance is not achieved.

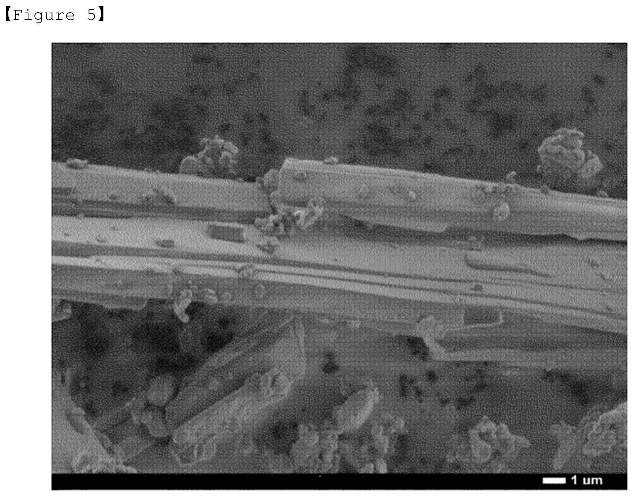

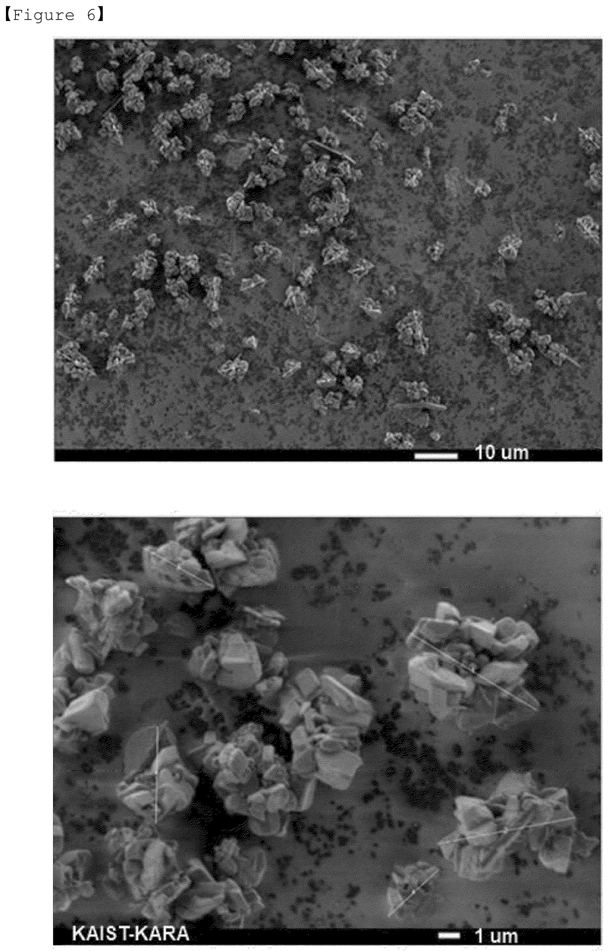

In addition, the molecular association in which axitinib is physically bonded according to the present invention may have an aspect ratio value of 0.3 to 1.0. That is, conventional general axitinib has an aspect ratio value of less than 0.3, and thus has an elongated rod-type shape as shown in Figure 5. In contrast, the molecular association in which axitinib is physically bonded according to the present invention has a physically bound structure of pure axitinib, and thus has a difference in that it exhibits the characteristic of having an aspect ratio value of 0.3 or more, and specifically has a relatively round shape as shown in Figure 6.

The molecular association in which axitinib is physically bonded according to the present invention may have an aspect ratio of 0.3 or more, 0.4 or more, 0.5 or more, 0.6 or more, 0.7 or more, 0.8 or more, 0.9 or more, and may have an aspect ratio of 1.0 or less, 0.9 or less, 0.8 or less.

In the present invention, the aspect ratio of a particle may be determined by measuring the length and thickness of the particle using any suitable measurement technique, preferably using a dynamic image analysis method performed according to the ISO 13322-2:2006 standard and calculating the aspect ratio from the measured dimensions of the particle as described above.

In addition, the molecular association in which axitinib is physically bonded according to the present invention may have the following solubility: a dissolution concentration at pH 1 of 3.0 mg/mL or more and a dissolution concentration at pH 2 of 0.1 mg/mL or more.

Specifically, the molecular association in which axitinib is physically bonded according to the present invention may have a dissolution concentration at pH 1 of 3.0 mg/mL or more, 3.5 mg/mL or more, 4.0 mg/mL or more, 4.3 mg/mL or more, and the upper limit may be, but is not particularly limited to, 10.0 mg/mL or less.

In addition, specifically, the molecular association in which axitinib is physically bonded according to the present invention may have a dissolution concentration at pH 2 of 0.1 mg/mL or more, 0.3 mg/mL or more, 0.5 mg/mL or more, 1.0 mg/mL or more, 1.5 mg/mL or more, 1.7 mg/mL or more, and the upper limit may be, but is not particularly limited to, 5.0 mg/mL or less.

Axitinib of the present invention may have a solubility that is 1.5 or 2 times higher than the solubility of the original axitinib itself.

### Method for producing axitinib of the present invention

Axitinib according to an embodiment of the present invention may be produced by applying shear stress to a solution containing axitinib or a salt of axitinib, which is a precursor of the structure.

The shear stress applied to the solution containing axitinib, which is a precursor of the structure, may be either mechanical shear stress or ultrasonic application.

The mechanical shear stress may be applied by passing the solution through a column filled with silica or a filter paper. Hereinafter, mechanical shear stress will be described in detail.

According to an embodiment of the present invention, the mechanical shear stress may be applied by passing a solution containing axitinib through a column filled with silica. When the solution containing axitinib passes through a column filled with silica or the like, the axitinib undergoes a very high shear stress as it passes through a physically narrow area.

The silica may be spherical or polygonal, but its shape is not limited.

The size of the silica may be 0.01 to 100 µm, preferably 0.1 to 10 µm, and more preferably 2.5 to 3.7 µm. When the size of the silica is less than 0.01 µm or excesses 100 µm, even if the solution containing axitinib passes through a column filled with silica, shear stress may not be applied, so that there may be no change in the structure.

A negative pressure of 0.1 bar to 1.0 bar or 0.2 bar to 0.9 bar may be applied to the bottom of the column filled with the silica. When the negative pressure applied to the bottom of the column filled with the silica is less than 0.1 bar, the time required for the solution containing axitinib to pass through the column is increased, so that the production time of axitinib according to the present invention may be delayed. In addition, when the negative pressure applied to the bottom of the column filled with the silica excesses 1.0 bar, the time required for the solution containing axitinib to pass through the column is reduced, so that the production time of axitinib according to the present invention may be shortened, but production costs may increase because additional pump equipment is required.

According to another embodiment of the present invention, the mechanical shear stress may be applied by passing the solution containing axitinib through one or more filter papers. When it passes through the one or more filter papers, the axitinib undergoes a very high shear stress as it passes through a physically narrow area.

The filter paper may be one filter paper or two or more filter papers. When the filter paper consists of two or more filter papers, the filter papers may be arranged in a stacked manner. When the filter paper consists of two or more filter papers, high shear stress may be provided compared to a single filter paper.

The pore size of the filter paper may be 0.1 to 5.0 microns or 0.3 to 4.5 microns. When the pore size of the filter paper is less than 0.1 micron, the amount of the solution containing axitinib passing through or filtering through the filter paper is too small, so that the production speed of axitinib according to the present invention may be reduced, and when the pore size of the filter paper excesses 5.0 microns, the solution containing axitinib simply passes through the filter paper, so that shear stress may not be effectively applied.

### [Best Mode]

Hereinafter, the present invention will be described in more detail through Examples of the present invention. It will go without saying that the present invention is not limited to these examples.

### [EXAMPLES]

### Example 1. Method for producing novel axitinib polymorph (SCAI-Form)

16.0 g of axitinib (Shilpa company, India) was dissolved in 16.0 kg of ethanol (94.5% ethanol, Samchun company) to prepare a dissolved solution of axitinib at a concentration of about 0.1%.

270 g of SYLOID 244FP (GRACE company, USA) was wetted with 4.32 kg of ethanol (94.5% ethanol, Samchun company), and a 1.0 µm paper filter was combined with a Nutsche filter with a diameter of 350 mm. A SYLOID 244FP column with a height of approximately 1.4 cm was prepared by pouring the SYLOID 244FP solution wetted with ethanol into the Nutsche filter.

1.08 kg of the prepared 94.5% ethanol was added onto the column using vacuum to strengthen the SYLOID 244FP column. The prepared dissolved solution of axitinib was added, and an additional 3.24 kg of 94.5% ethanol was passed through the column to recover the axitinib remaining in SYLOID 244FP. In this case, the weight of the axitinib effluent was approximately 21.86 kg.

The axitinib effluent was filtered using a 0.45 µm PVDF membrane filter and concentrated to a concentrate concentration of 3.0 mg/g using a rotary vacuum concentrator. Once concentration was completed, the axitinib concentrate was filtered using a 0.2 µm PVDF membrane filter.

53.0 kg of purified water was added to a 100 L reactor, and the prepared axitinib concentrate was slowly added while rapidly stirring the purified water. After the addition was completed, additional stirring was performed for 30 minutes. The mixed solution was filtered using a 1.0 µm paper filter.

The filtered cake was vacuum decompressed for 30 minutes and dried using nitrogen for 2 hours. In addition, it was dried in a vacuum oven at 25 °C for 38 hours to obtain 14.07 g (yield of 88%) of axitinib as a white powder.

### Comparative Example 1. Axitinib API

It is a commercially available axitinib substance (Shilpa company, India).

### [Experimental Examples]

### Experimental Example 1. X-ray diffractometry (XRD) of novel axitinib polymorph (SCAI-Form)

The reagent was placed in a sample holder, pressed with a glass rod, and filling molded into the filling part, and it shows a crystal form when tested according to the X-ray powder diffraction method among the general test methods in the "Korean Pharmacopoeia."

Table 1 below shows the operating conditions.

**[Table 1]**

| Conditions | Actual measuring conditions | Conditions | Actual measuring conditions |
|---|---|---|---|
| X-ray tube | Copper K2 a | 2 Theta start | 5° |
| Counter | Scintillation counter | 2 theta end | 60° |
| Voltage | 40 kV | Speed | 2°/min |
| Current | 15 mA | Scane type | Vertical type |
| Wavelength | 1.5406 Å | Scane mode | continuous |
| Detector | D/tex Ultra2 | Divergence slit | 1.25°DS/21HS |

X-ray powder diffraction patterns of various polymorphic forms were performed on a Rigaku Miniflex600 using copper radiation (CuKα, wavelength of 1.5406 Å). The voltage and current of the tube were set to 40 kV and 15 mA, respectively. The divergence and scattering slits were set to 8.0 mm and the receiving slit was set to 13.0 mm. Diffracted radiation was detected with D/teX Ultra2. Theta-2 theta continuous scan was used at 2.0°/min (1 sec/0.03° step) from 3.0° to 60° 2θ. Alumina standards were analyzed to confirm instrument alignment. Data was collected and analyzed using SmartLab Studio II.

X-ray powder diffraction patterns were measured on a Rigaku Miniflex 600 using copper radiation (CuKα, wavelength of 1.54056 Å). The voltage and current of the tube were set to 40 kV and 15 mA, respectively. The divergence and scattering slits were set to 8.0 mm and the receiving slit was set to 13.0 mm. Diffracted radiation was detected with D/teX Ultra2. Theta-2 theta continuous scan was used at 2.0°/min (1 sec/0.03°) from 3.0° to 60° 2θ. Alumina standards were analyzed to confirm instrument alignment. Data was collected and analyzed using SmartLab Studio II.

Table 2 below shows the XRD results, i.e., 2 theta and relative intensity of axitinib, which is the API, and SCAI-Form, which is the molecular association of axitinib according to the present invention.

**[Table 2]**

| API | | SCAI-Form (AXTEN03A) | |
|---|---|---|---|
| Angle | Relative intensity (%) | Angle | Relative intensity (%) |
| 8.19 | 102.70 | 8.84 | 53.2 |
| 11.92 | 4.23 | 11.99 | 133.1 |
| 14.74 | 3.93 | 14.60 | 26 |
| 15.34 | 20.13 | 15.24 | 51.9 |
| 15.56 | 43.41 | 15.70 | 76.6 |
| 17.45 | 57.19 | 17.71 | 27.6 |
| 19.39 | 4.72 | 19.33 | 58.1 |
| 20.69 | 8.41 | 20.65 | 58.8 |
| 21.40 | 9.21 | 21.70 | 85.1 |
| 23.29 | 44.46 | 23.23 | 53.4 |
| 24.01 | 55.39 | 24.19 | 38 |
| 25.96 | 100 | 24.99 (25.0) | 100 |
| 26.24 | 3.75 | 26.32 (26.3) | 38.8 |
| 27.78 | 8.13 | 27.59 | 11.9 |

### Experimental Example2. Differential scanning calorimetry (DSC) of novel axitinib polymorph (SCAI-Form)

It was measured using a heating program (Table 3) in a differential scanning calorimeter device. In this case, the sample amount is recommended to be 4.0 mg or less, the environment within the device is maintained as nitrogen, and the nitrogen flow rate is 10 mL/min.

**[Table 3]**

| Rate | Target | Hold | Record |
|---|---|---|---|
| 20 °C/min | 100 °C | 20 min | |
| 10 °C/min | 30 °C | 5 min | - |
| 10 °C/min | 250 °C | 5 min | √ |

Table 4 below shows the peak temperature and ΔH of axitinib, which is the API, and SCAI-Form, which is the molecular association of axitinib according to the present invention.

**[Table 4]**

| | API | SCAI-Form (AXTEN03A) |
|---|---|---|
| Peak temp. (°C) | 212.5 | 220.4 |
| | 220.6 | |
| H (J/g) | 129.1 | 131.7 |

### Experimental Example 3. Scanning Electron Microscopy (SEM) of novel axitinib polymorph (SCAI-Form)

### Measuring conditions

Powder samples were placed on carbon tape fixed to aluminum stubs. The samples were scanned in FE-SEM using JSM-IT800, Jeol. Images were acquired at an acceleration voltage of 1.00 kV using a secondary electron detector.

The crystal structures of Axitinib API and SCAI-Form were analyzed using the above measurement method.

Axitinib API has the shape of a square bar like a tree splinter (see Figure 5)

On the other hand, the novel axitinib polymorph (SCAI-Form) of the present invention has a non-sharp (round) polyhedral shape (see Figure 6).

In addition, it could be confirmed that Axitinib API had an aspect ratio much smaller than 0.3, as shown in Figure 5, and it could be confirmed that it had an aspect ratio close to 1, as shown in Figure 6.

Among the images in Figure 6, the lower image is an enlarged version of the upper image, and the maximum diameter of the molecular association according to the present invention could be measured as shown in Table 5 below.

**[Table 5]**

| **Classification** | **Diameter (length, µm)** |
|---|---|
| **Average value** | 6.68 |
| **Minimum value** | 3.29 |
| **Maximum value** | 12.84 |

### Experimental Example 4. Solubility of new axitinib polymorph (SCAI-Form)

Method for measuring solubility at pH 1: Stirred for 15 minutes at a concentration of 5 mg/mL, and then filtered, diluted 10 times with DW, and analyzed. (The value calculated as [measured value x 10] is described in the results)

Method for measuring solubility at pH 2: Stirred for 15 minutes at a concentration of 1 mg/mL, and then filtered, analyzed, and shown in Table 6.

**[Table 6]**

| **Classification** | **Batch No.** | **Dissolution concentration at pH 1 (mg/mL)** | **Dissolution concentration at pH 2 (mg/mL)** |
|---|---|---|---|
| **API** | **Axitinib API (Shilpa)** | 2.661 | 0.073 |
| **SCAI-Form** | **AXTEN03A** | 4.363 | 0.171 |

From the above, it can be seen that the solubility of SCAI-Form is about twice higher than that of API.

## Claims

1. A molecular association in which axitinib is physically bonded,
wherein the X-ray powder diffraction spectrum of the molecular association has X-ray diffraction peaks at diffraction angles 2θ of 24.99°±0.1° and 26.32°±0.1°.

2. The molecular association according to claim 1, **characterized in that** the molecular association has a differential scanning calorimetry (DSC) profile with a glass transition at a single endothermic temperature of 220.4±2.0 °C when measured under DSC conditions of 10 °C/min heating rate, 99.999% N2 and 30 to 250 °C.

3. The molecular association according to claim 1, **characterized in that** the molecular association has an aspect ratio value of 0.3 to 1.0.

4. The molecular association according to claim 1, **characterized in that** the molecular association has an average particle diameter of 2 to 15 µm.

5. The molecular association according to claim 1, **characterized in that** the molecular association has the following solubility:
a dissolution concentration at pH 1 of 3.0 mg/mL or more, and
a dissolution concentration at pH 2 of 0.1 mg/mL or more.
